# EUROPEAN PATENT SPECIFICATION

(11) **EP 3 122 236 B1**
(45) Date of publication and mention of the grant of the patent: **16.05.2018**
(21) Application number: 14712311.1
(22) Date of filing: 26.03.2014
(51) Int. Cl.: A61B 5/00, G08B 29/10, G08B 29/12, G08B 21/02, H04R 1/02, H04R 29/00, A61B 5/0476

(54) **BIO-ELECTRICAL SIGNAL MONITOR WITH TWO SPEAKERS**
BIO-ELEKTRISCHE SIGNAL ÜBERWACHUNG MIT ZWEI LAUTSPRECHERN
SURVEILLANCE DES SIGNAUX BIOÉLECTRIQUE AVEC DEUX HAUT-PARLEURS

(43) Date of publication of application: 01.02.2017
(73) Proprietor: T&W Engineering A/S, 3540 Lynge (DK)
(72) Inventor: LARSEN, Tina Ahlberg, DK-3540 Lynge (DK); JENSEN, Flemming Dahl, DK-3460 Birkerod (DK); CLAUSEN, Bent, DK-3540 Lynge (DK); CHRISTENSEN, Erik Skov, DK-3540 Lynge (DK); KILSGAARD, Soren, DK-3540 Lynge (DK); JENSEN, Morten Holm, DK-3520 Farum (DK); FRIIS, Lars, DK-3540 Lynge (DK)
(74) Representative: Bastue, Jens
(86) International application number: PCT/EP2014/056010
(87) International publication number: WO 2015/144214

(56) References cited:
- WO-A1-2007/041329
- WO-A1-2010/149157
- US-A1- 2011 135 120

## Description

The present invention relates to a monitor for monitoring bio-electrical signals from a person. The invention relates more particularly to a personal wearable monitor for monitoring a bio-electrical signal from a person. This monitor comprises a speaker for providing information to the person.

Bio-electrical signals are here understood to be electrical potential differences across a tissue, organ or cell system. The best known examples are Electrocardiogram signals (ECG) and Electroencephalogram signals (EEG). By a personal wearable monitor is meant a monitor that is convenient in wearing, preferably also over an extended interval of time, e.g. several months or years, where the person can live a normal life without having to pay more attention to the monitor than necessary with a pair of glasses or a hearing aid. The monitoring may be for purposes of surveillance of a condition of the person and for providing some kind of alarm or information in case predetermined conditions are met. The monitor may also be applied for collection of data for further analysis, e.g. for diagnostic purposes or for research use.

Monitors for measuring EEG signals are known from e.g. US 8118741 B2 or WO-A2-2007/150003. US 8241221 B2 discloses an ECG monitor system adapted for providing an alarm if a stroke is detected.

An example of monitoring bio-electrical signals is the recording and analysing of an EEG signal for various diagnostic purposes. WO-A1-2006/047874 describes measurement of brain waves particularly for detecting the onset of an epileptic seizure. Also WO 2010/149157 A1 discloses an EEG monitoring apparatus adapted for warning the person carrying the EEG monitoring apparatus against e.g. epileptic seizures and comprising an audio generator and a loudspeaker. EEG monitors may also be applied for surveillance of persons having diabetes, where low blood sugar levels may cause hypoglycaemic attacks. A system for surveillance of the EEG signal where changes may indicate an imminent hypoglycaemic attack is disclosed in WO-A-2006/066577.

A problem in the known bio-electrical signal monitors for detecting and informing about an upcoming seizure, is that the speaker in such a monitor is a mechanical component with an open connection to the open air or to the ear canal, and therefore subject to a risk of failure. A failure may also be caused by a bad soldering or by corrosion of a wire or a soldering. If the speaker has failed the processor of the monitor will not know, and if the monitor detects a condition of an upcoming seizure and sends an alarm signal to the speaker, the person who should have been warned may not know before it is too late.

It has been found that the above problem can be solved by a personal wearable monitor for monitoring a bio-electrical signal from a person wherein the monitor is adapted for detecting an upcoming seizure, and for providing an acoustical information signal. The monitor is provided with a first speaker for providing the acoustic information signal, and with a second speaker adapted for functioning as a microphone (and as a back-up speaker) in testing if said first speaker is capable of providing a sound. The monitor is adapted for providing a notification in the event that said second speaker does not detect the sound generated from said first speaker.

The generated sound mentioned here may be an acoustical information signal or it may be a test sound. The second speaker which is adapted to function as microphone will also function as speaker, at least as a backup speaker in the case of the first speaker not delivering a sound.

The implication of the speaker being capable of delivering or providing a sound is, that the speaker is operable to generate the sound and that the speaker is not blocked from delivering the sound to the close surroundings, e.g. that the sound tube is not blocked.

The advantage of the solution is that the monitor of the invention on its own will be able to detect a malfunctioning speaker, and to notify about this malfunctioning speaker, and to remedy the problem until the speaker has been repaired or changed.

In an embodiment of the monitor, the monitor is adapted for providing a verification signal or a test sound through the first speaker, and the verification signal is provided at specific time intervals and in a specific frequency range. This verification signal is a test sound with the purpose of testing the first speaker. This has the advantage that a malfunction of the speaker may be recognized within short time, and possibly before it is necessary to provide an essential information, e.g. about an upcoming seizure to the person wearing the monitor.

In a further embodiment, at least one of the first or the second speaker is adapted for being applied as microphone for picking-up or measuring the background sound level. This gives the signal processor of the monitor the possibility of selecting a sound level which is easily discernible over the background noise for any acoustic information provided.

In an embodiment, the monitor comprises a signal processor adapted for analyzing said bio-electrical signal in order to identify or predict predetermined biological incidents in said person. This offers the possibility of providing the person wearing the monitor with an alarm or some kind of notification.

In a further embodiment, the monitor comprises a decision means adapted to decide when information is to be presented to said person. The decision means could be a classifier basing its classification on an empirical model.

In a further embodiment, the acoustic information signal of the monitor is in the form of a spoken message. This makes it possible to provide more specific information, and e.g. to give guidance to the person.

In a further embodiment, the monitor is arranged at the ear, which makes it easy to provide an acoustic information signal to the person wearing the monitor. In case the bio-electrical signal is an EEG signal, a position adjecent the ear is also advantageous, since the ear region offers good EEG pick-up positions.

In a further embodiment, the acoustical information signal from the monitor is provided to the ear canal of said person. This may be through a sound tube secured in the ear canal by an ear tip. This makes it easier for the person to hear an acoustic information signal.

In a further embodiment, the first and second speakers of the monitor are arranged to share the same sound tubing to guide the sound. This will save space.

In a further embodiment, the first and second speakers are arranged together as an integral unit i.e. one unit. This will also save space and will simplify logistics in relation to manufacturing.

In a further embodiment, the monitor is adapted for testing if said second speaker is capable of delivering a sound wherein said first speaker is adapted for functioning as a microphone. With this function the monitor will be able to prepare for the situation that the second speaker, which also may have the function as a back-up speaker, does not function properly when needed. If the second speaker does not function when tested, a notification should be provided. If any defects is found for any one of the speakers delivering sound, and it becomes necessary to provide an acoustical information signal, this could be done by the application of both speakers simultaneously, preferably making sure that the two speakers are in phase.

In a second aspect, the invention is directed to a method for monitoring a bio-electrical signal from a person, and for detecting an upcoming seizure by analysis of this signal. The method comprises the four steps: 1) providing an acoustical information signal to the person in the event that a condition of an upcoming seizure is detected, 2) providing the information signal by a first speaker, 3) testing if this first speaker is capable of providing a sound by application of a second speaker adapted for functioning as a microphone, and 4) providing a notification (e.g. by the second speaker) in the event that the second speaker does not detect the sound from the first speaker.

Some embodiments will now be explained in further detail with reference to the figures.
Figure 1 illustrates a block diagram of a bio-electrical signal monitor;
Figure 2 illustrates an embodiment where an EEG monitoring system is arranged at the ear of a person with an implant comprising electrodes arranged subcutaneously in the area behind the ear and with an external part comprising speakers;
Figure 3 illustrates how two microphones can be arranged in a block in a part of a bio-electrical signal monitor;
Figure 4 illustrates the monitor part of figure 3 with further components;
Figure 5 illustrates one block with two microphones; and
Figure 6 illustrates the block of figure 5, but seen from a different angle.

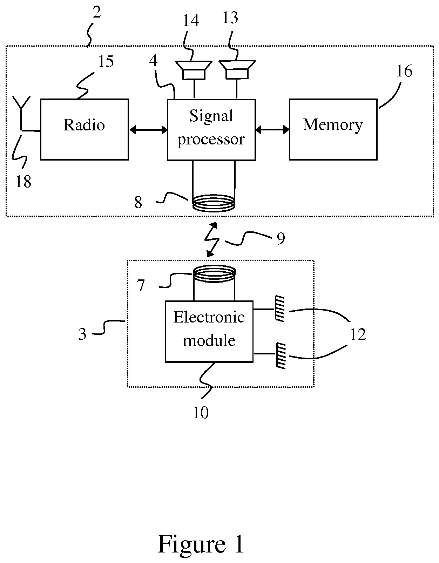

Figure 1 shows an example of the general layout of a monitor for monitoring bio-electrical signals. The monitor is in this example in two parts. One is an electrode part 3 comprising electrodes 12 for measuring or capturing bio-electrical signals such as EEG or ECG, and comprising an electronic module 10 for preprocessing the bio-electrical signals and sending, e.g. by a coil 7, this signal to the other part of the monitor. This other part is in this example a processor part 2 comprising a signal processor 4 for detecting a condition of an upcoming seizure from the monitored bio-electrical signal.

The two parts are in this example interconnected by an inductive link 9 established by the coil 7 in the electrode part 3, and a co-aligned coil 8 in the processor part 2. With this coupling the electrode part 3 may be implanted, e.g. with the coil 7 placed subcutaneous for easy alignment with the coil 8, which is arranged external to the skin. Thereby, the advantages of an implant, such as good electrical contact between the electrodes and the tissue, can be combined with the advantages of having the acoustic transducers in the open air, i.e. better sound quality. Another advantage is that power can be supplied from the external processor part, which will usually comprise a battery, and to the implanted electrode part 3, through the inductive link 9.

If the electrode part 3 is adapted for being arranged external on the skin, then the inductive link could be replaced by a wired connection, or by a radio connection. Also, the processor part 2 and the electrode part 3 may be built into the same housing, e.g. with the electrodes arranged external on this housing, or as separate pads with wiring to the housing.

The electrode part 3 will be arranged to submit the bio-electrical signal to the signal processor 4 in the processor part 2. Preferably, analogue to digital conversion will take place in the electronic module 10 of the electrode part 3. The signal processor 4 is arranged for continuous analysis of the bio-electrical signal and is adapted for identifying or predicting predetermined biological incidents in the person wearing the monitor based on said analysis. Or the signal processor 4 is adapted for identifying a condition e.g. where the experience says that there is a risk of a biological incidence.

The analysis of the signal processor 4 may be based on algorithms developed from large amounts of data, i.e. an empirical algorithm. The signal processor preferably comprises a decision part adapted to decide when information is to be presented to said person. The decision part may comprise a classifier, classifying each sample of bio-electrical signal, where each sample represents a given time, e.g. 1 second. E.g. each sample could be classified into one of two groups: one where the risk for an upcoming seizure is present and one where the risk is insignificant. The classification may be based on empirical data.

In the example of figure 1, two speakers 13, 14 are arranged as part of the processor part 2. At least one of these speakers is adapted for providing an acoustical information signal. This information signal is often provided to the person being monitored, but could also be provided to other persons. The information signal can be in the form of an alarm sound, such as a beep sound, a spoken message or some other sound. Often, both speakers are adapted for being able to provide an acoustical information signal.

At least one of the speakers, e.g. the second speaker 14, is adapted for functioning as a microphone in testing if the other speaker, the first speaker 13, is capable of generating a sound. This provide a safety fall-back operation in order to make sure that the monitor will be able to provide an acoustical information signal, e.g. an alarm, in the event that a condition of an upcoming seizure is identified. The signal processor 4 may provide a test signal to the first speaker 13, e.g. at regular time intervals. The second speaker 14 is then set up as microphone to detect the acoustical signal from the first speaker 13. In case the second speaker 14 does not detect any signal from the first speaker 13, or only detects insufficient signal level, the monitor is adapted for providing a notification that some kind of maintenance or repair is needed.

If it is detected that the first speaker 13 does not function correctly, any acoustical information signal, e.g. related to an upcoming seizure, can be provided through the second speaker 14, e.g. until replacement or repair of the first speaker has been performed.

The second speaker 14 may also provide an acoustic alarm or message informing that the first speaker is not functioning properly.

The second speaker 14 can be used for controlling specific test sounds generated by the first speaker 13. The second speaker can also be used for testing if an acoustic information signal is actually provided by the first speaker 13 and, in the case that the acoustic information signal is not delivered by the first speaker 13 at the time where it should have been given; the second speaker 14 will provide the acoustic information signal instead.

The monitor may be set up for testing also if the second speaker 14 is able to provide a test signal if this should be necessary, e.g. as back-up for a mal-functional first speaker 13. Test of the second speaker 14 could then be performed by the application of the first speaker 13 as microphone. In case the second speaker is found not to be functioning properly, a notification should be given.

As shown in figure 1 the processor part 2 may also comprise other components. An example of this is a radio 15 with an antenna 18 for wireless communication with remote units, such as a mobile phone or computer. This may be applied for notifying about a malfunctioning speaker. A memory which may be applied for storage of sequences of bio-electrical signals may be part of a monitor. The memory may also comprise a library of speech messages for being used as different acoustic information signals. Further to this the processor part will usually comprise a power supply, often in the form of a battery. If the processor part 2 and the electrode part 3 are arranged in the same housing, some of the components may be arranged independently of these two parts.

Often the two speakers 13, 14 will be of the same type. Several types of speakers, or receivers, may be applied. One example is the Receiver 4100 from Sonion A/S. This type of speaker could also be applied in hearing aids, where they are called receivers. The speakers will preferably be arranged with separate wiring, in order for the signal processor 4 to be able to access them individually.

In the embodiment where a test sound is provided at specific time intervals, the time intervals could e.g. be in the range once every 0.5 to 5 hours, preferably once every 1 to 2 hours. The frequency of the test sound could e.g. be in the range 1 to 6 kHz, preferably around 3 kHz, where the sensitivity of a speaker used as microphone is often high. It will be possible to play the test sound at a low sound level, in order not to bother the person being monitored.

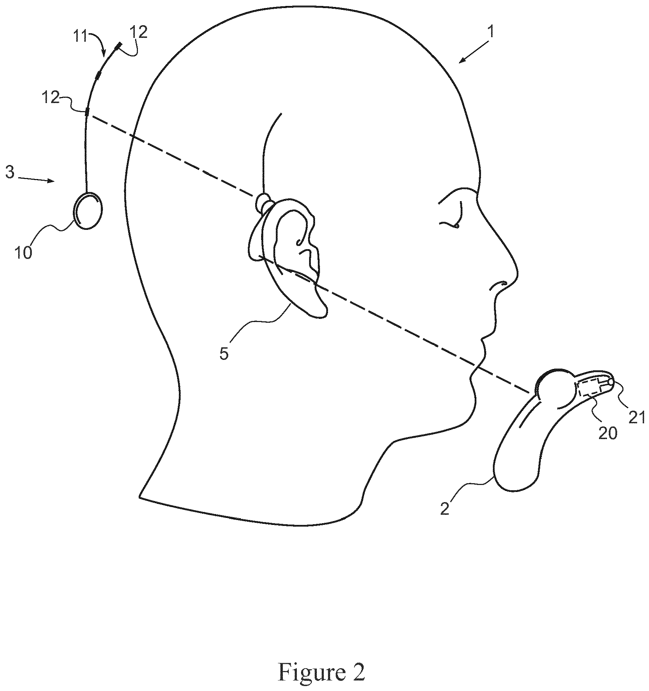

Figure 2 shows an example of a practical implementation of how a monitor may be arranged at the head 1 of the person to be monitored. The monitor is here arranged at the ear 5 or in connection with the ear or behind the ear. In figure 2 the processor part 2 of the monitor is arranged behind the ear 5 external to the skin. The electrode part 3 is implanted subcutaneous, also behind the ear. The electrode part 3 comprises an electronic module 10 arranged in a hermetically sealed housing with a coil 7. A wire 11 with three active and separate electrode points 12 is extending from this housing. The processor part 2 should then be arranged such that the coil 8 is aligned in relation to the coil 7 of the electrode part 3.

In figure 2 a casing 20 comprising both speakers 13, 14 is indicated inside the processor part 2. This casing 20 has an internal conduit or manifold (not shown) for connecting sound outputs of the speakers with a sound opening 21. A sound tube (not shown) could be applied for guiding the sound from sound opening 21 into the ear canal of the person. An alternative could be to arrange the speakers 13, 14 external to the processor part housing connected with wires to the processor part 2. The speakers could be arranged directly in the ear canal. In another example, also the electrodes could be arranged in the ear canal.

Even if the first speaker 13 functions well, the sound opening 21 or a sound tube guiding the sound to the ear canal, may be mechanically blocked, thereby blocking the sound. This can also be detected by playing a sound through the first speaker 13 and detecting the sound level reached by the second speaker 14. Sound may be provided through separate sound tubes from each speaker. There should then be a good chance that if one is blocked the other will still be open for sound transmission. Preferably, only one common sound tube is applied.

The first speaker 13 (or the second speaker 14), may also be applied as microphone for detecting the general background noise level at any time. This can be applied for deciding the sound level of any acoustic information signal or notification, such that it is easily discernible over background noise.

If the person being monitored does not respond to a notification about a condition of an upcoming biological incidence, such as hypoglycemia or an epileptic attack, the sound level could be increased, and eventually both speakers could be applied for providing the notification in order to obtain the loudest possible acoustical information signal.

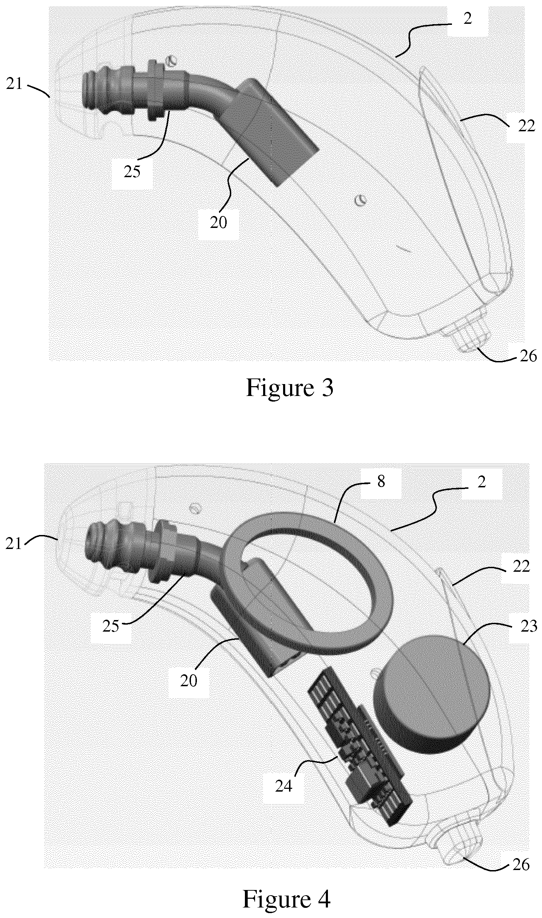

Figure 3 shows an example of the processor part 2 of a monitor, which is adapted for being arranged external on the skin surface, e.g. behind the ear as illustrated in figure 2. The housing of the processor part 2 is illustrated with a battery door 22. Also a sound opening 21 is illustrated. A speaker block 20 is arranged inside the housing of the processor part 2 and comprises the two speakers 13, 14. The two speakers could also be arranged as separate units, but building them together as one unit will save space and make manufacturing of the processor part 2 easier.

Also a pushbutton 26 is illustrated in figure 3. The person wearing the monitor may use this button 26 for the acknowledgement that a notification have been heard and e.g. complied with. Such an acknowledgement could also, in some set-ups of the monitor, be provided through an external device (e.g. a mobile phone or a watch) which is wirelessly connected to the monitor. This could be relevant especially when the notification is provided through such an external device.

The speaker block 20 is connected to the sound outlet 21 through a tubing 25. From the sound outlet 21 the acoustical information signal may be guided into or towards the ear canal of the person being monitored by the use of a sound tube (not shown).

By having the two speakers connected to the same sound tubing system, and being able to set one receiver up as microphone, it is possible to detect changes in the acoustic impedance of the sound tubing system. Thereby, it will be possible to see if the sound tubing is being filled up with dirt or earwax, and to provide a warning before the sound tubing is completely blocked.

Figure 4 shows the example of figure 3 with further components shown in the housing of the processor part 2. In addition to the components shown in figure 3, figure 4 includes a coil 8 for transfer of data and power between the processor part 2 and the electrode part 3. Also, a battery 23 is shown, as well as an electronic circuit 24 comprising the signal processor 4 and possibly also the memory 16 and parts of the radio 15 (see figure 1).

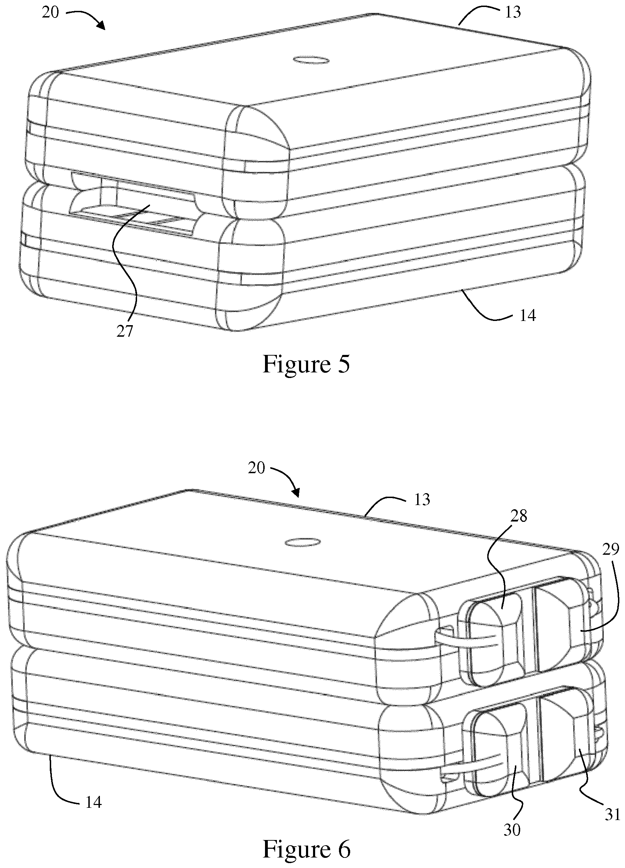

Figure 5 shows an example of a speaker block 20 with two different speakers 13, 14 placed in close connection and with one common sound outlet 27.

Figure 6 shows the speaker block of figure 5 seen from a different angle such that the electrical terminals 28, 29, 30, 31 for connecting each speaker 13, 14 with the signal processor 4 becomes visible.

## Claims

1. A personal wearable monitor for monitoring a bio-electrical signal from a person, said monitor being adapted for detecting a condition of an upcoming seizure, and for providing an acoustical information signal, said monitor being provided with
- a first speaker (13) for providing said information signal, and
- a second speaker (14) adapted for functioning as a microphone in testing if said first speaker (13) is capable of delivering a sound,
and said monitor being adapted for providing a notification in the event that said first speaker (13) is not capable of delivering sound.

2. The monitor according to claim 1, adapted for providing said notification as acoustical information by said second speaker (14).

3. The monitor according to claim 1 or 2, adapted for providing a verification signal through said first speaker (13), wherein the verification signal is provided at specific time intervals and in a specific frequency range.

4. The monitor according to any one of the preceding claims, wherein at least one of said first or second speakers (13; 14) is adapted for being applied as a microphone for picking-up the background sound level.

5. The monitor according to any one of the preceding claims, comprising a signal processor (4) adapted for analyzing said bio-electrical signal in order to identify or predict conditions of predetermined biological incidents in said person.

6. The monitor according to any one of the preceding claims, comprising a decision means adapted to decide when information is to be presented to said person.

7. The monitor according to any one of the preceding claims, wherein said acoustical information signal is in the form of a spoken message or a beep.

8. The monitor according to any one of the preceding claims, wherein said monitor is arranged at the ear.

9. The monitor according to any one of the preceding claims, wherein said acoustical information signal is provided to the ear canal of said person.

10. The monitor according to any one of the preceding claims, wherein the first and second speakers (13, 14) are arranged to share a common sound tubing to guide the sound.

11. The monitor according to any one of the preceding claims, wherein the first and second speakers (13, 14) are arranged together as an integral unit.

12. The monitor according to any one of the preceding claims, adapted for testing if said second speaker (14) is capable of delivering a sound wherein said first speaker (13) being adapted for functioning as a microphone.

13. The monitor according to any one of the preceding claims, comprising a radio (15) adapted for notifying a remote unit wirelessly if a speaker is not capable of delivering a sound.

14. A method for monitoring a bio-electrical signal from a person, and for detecting a condition of an upcoming seizure by analysis of said signal, said method comprising
- providing an acoustical information signal to said person in the event that a condition of an upcoming seizure is detected,
- providing said information signal by a first speaker (13),
- testing if said first speaker (13) is capable of delivering a sound by application of a second speaker (14) as a microphone, and
- providing a notification in the event that said second speaker (14) does not detect said sound from said first speaker (13).

15. The method according to claim 14, wherein said notification is provided as acoustical information by said second speaker (14).

## Patentansprüche

1. Persönlicher tragbarer Monitor zum Überwachen eines bioelektrischen Signals von einer Person, wobei der Monitor eingerichtet ist zum Erkennen eines Zustandes eines bevorstehenden Anfalls und zum Bereitstellen eines akustischen Informationssignals, wobei der Monitor mit Folgendem versehen ist:
- einem ersten Lautsprecher (13) zum Bereitstellen des Informationssignals und
- einem zweiten Lautsprecher (14), der eingerichtet ist zum Funktionieren als ein Mikrofon beim Prüfen, ob der erste Lautsprecher (13) dazu in der Lage ist, einen Schall zu liefern,
und wobei der Monitor eingerichtet ist zum Bereitstellen einer Benachrichtigung in dem Fall, dass der erste Lautsprecher (13) nicht dazu in der Lage ist, Schall zu liefern.

2. Monitor nach Anspruch 1, der eingerichtet ist zum Bereitstellen der Benachrichtigung als akustische Information durch den zweiten Lautsprecher (14).

3. Monitor nach Anspruch 1 oder 2, der eingerichtet ist zum Bereitstellen eines Prüfsignals durch den ersten Lautsprecher (13), wobei das Prüfsignal in spezifischen Zeitabständen und in einem spezifischen Frequenzbereich bereitgestellt wird.

4. Monitor nach einem der vorhergehenden Ansprüche, wobei wenigstens einer vorn dem ersten oder dem zweiten Lautsprecher (13; 14) dafür eingerichtet ist, als ein Mikrofon zum Aufnehmen des Hintergrund-Geräuschpegels angewendet zu werden.

5. Monitor nach einem der vorhergehenden Ansprüche, der einen Signalprozessor (4) umfasst, der eingerichtet ist zum Analysieren des bioelektrischen Signals, um Zustände vorbestimmter biologischer Vorgänge bei der Person zu identifizieren oder vorherzusagen.

6. Monitor nach einem der vorhergehenden Ansprüche, der ein Entscheidungsmittel umfasst, das dafür eingerichtet ist, zu entscheiden, wann der Person Informationen dargeboten werden sollen.

7. Monitor nach einem der vorhergehenden Ansprüche, wobei das akustische Informationssignal die Form einer gesprochenen Mitteilung oder eines Pieptons hat.

8. Monitor nach einem der vorhergehenden Ansprüche, wobei der Monitor am Ohr angeordnet ist.

9. Monitor nach einem der vorhergehenden Ansprüche, wobei das akustische Informationssignal am Gehörgang der Person bereitgestellt wird.

10. Monitor nach einem der vorhergehenden Ansprüche, wobei der erste und der zweite Lautsprecher (13, 14) dafür angeordnet sind, eine gemeinsame Schallröhre zu teilen, um den Schall zu leiten.

11. Monitor nach einem der vorhergehenden Ansprüche, wobei der erste und der zweite Lautsprecher (13, 14) zusammen als eine integrale Einheit angeordnet sind.

12. Monitor nach einem der vorhergehenden Ansprüche, der eingerichtet ist zum Prüfen, ob der zweite Lautsprecher (14) dazu in der Lage ist, einen Schall zu liefern, wobei der erste Lautsprecher (13) eingerichtet ist zum Funktionieren als ein Mikrofon.

13. Monitor nach einem der vorhergehenden Ansprüche, der ein Funkgerät (15) umfasst, das eingerichtet ist zum drahtlosen Benachrichtigen einer entfernten Einheit, falls ein Lautsprecher nicht dazu in der Lage ist, einen Schall zu liefern.

14. Verfahren zum Überwachen eines bioelektrischen Signals von einer Person und zum Erkennen eines Zustandes eines bevorstehenden Anfalls durch Analyse des Signals, wobei das Verfahren Folgendes umfasst:
- Bereitstellen eines akustischen Informationssignals in dem Fall, dass ein Zustand eines bevorstehenden Anfalls erkannt wird,
- Bereitstellen des Informationssignals durch einen ersten Lautsprecher (13),
- Prüfen ob der erste Lautsprecher (13) dazu in der Lage ist, einen Schall zu liefern, durch Anwendung eines zweiten Lautsprechers (14) als ein Mikrofon und
- Bereitstellen einer Benachrichtigung in dem Fall, dass der zweite Lautsprecher (14) den Schall von dem ersten Lautsprecher (13) nicht erfasst.

15. Verfahren nach Anspruch 14, wobei die Benachrichtigung als akustische Information durch den zweiten Lautsprecher (14) bereitgestellt wird.

## Revendications

1. Moniteur personnel portable pour surveiller un signal bioélectrique issu d'une personne, ledit moniteur étant adapté pour détecter un état d'une crise d'épilepsie prochaine et pour fournir un signal d'information acoustique, ledit moniteur étant pourvu :
- d'un premier haut-parleur (13) pour fournir ledit signal d'information et
- d'un second haut-parleur (14) adapté pour fonctionner comme microphone lorsque l'on teste si ledit premier haut-parleur (13) est capable de délivrer un son,
et ledit moniteur étant adapté pour fournir une notification dans le cas où ledit premier haut-parleur (13) n'est pas capable de délivrer un son.

2. Moniteur selon la revendication 1, adapté pour fournir ladite notification sous la forme d'informations acoustiques par ledit second haut-parleur (14).

3. Moniteur selon la revendication 1 ou 2, adapté pour fournir un signal de vérification via ledit premier haut-parleur (13), dans lequel le signal de vérification est fourni à intervalles de temps spécifiques et dans une plage spécifique de fréquences.

4. Moniteur selon l'une quelconque des revendications précédentes, dans lequel au moins l'un desdits premier ou second haut-parleurs (13 ; 14) est adapté pour être utilisé comme microphone pour recueillir le niveau sonore de fond.

5. Moniteur selon l'une quelconque des revendications précédentes, comprenant un processeur de signal (4) adapté pour analyser ledit signal bioélectrique afin d'identifier ou de prédire des conditions d'incidents biologiques prédéterminés chez ladite personne.

6. Moniteur selon l'une quelconque des revendications précédentes, comprenant un moyen de décision adapté pour décider quand des informations doivent être présentées à ladite personne.

7. Moniteur selon l'une quelconque des revendications précédentes, dans lequel ledit signal d'information acoustique se présente sous la forme d'un message parlé ou d'un bip.

8. Moniteur selon l'une quelconque des revendications précédentes, dans lequel ledit moniteur est agencé dans l'oreille.

9. Moniteur selon l'une quelconque des revendications précédentes, dans lequel ledit signal d'information acoustique est fourni au canal auriculaire de ladite personne.

10. Moniteur selon l'une quelconque des revendications précédentes, dans lequel le premier et le second haut-parleur (13, 14) sont agencés pour partager un tube sonore commun pour guider le son.

11. Moniteur selon l'une quelconque des revendications précédentes, dans lequel le premier et le second haut-parleur (13, 14) sont agencés conjointement sous la forme d'une unité intégrée.

12. Moniteur selon l'une quelconque des revendications précédentes, adapté pour tester si ledit second haut-parleur (14) est capable de délivrer un son, dans lequel ledit premier haut-parleur (13) est adapté pour fonctionner comme microphone.

13. Moniteur selon l'une quelconque des revendications précédentes, comprenant une radio (15) adaptée pour notifier à une unité distante sans fil si un haut-parleur n'est pas capable de délivrer un son.

14. Procédé de surveillance d'un signal bioélectrique venant d'une personne et pour détecter un état d'crise d'épilepsie prochaine par analyse dudit signal, ledit procédé comprenant les étapes consistant à :
- fournir un signal d'information acoustique à ladite personne dans le cas où un état d'une crise d'épilepsie prochaine est détecté,
- fournir ledit signal d'information par un premier haut-parleur (13),
- tester si ledit premier haut-parleur (13) est capable de délivrer un son par application du second haut-parleur (14) comme microphone et
- fournir une notification dans le cas où ledit second haut-parleur (14) ne détecte pas ledit son provenant dudit premier haut-parleur (13).

15. Procédé selon la revendication 14, dans lequel ladite notification est fournie sous la forme d'informations acoustiques par ledit second haut-parleur (14).
